# EUROPEAN PATENT SPECIFICATION

(11) **EP 1 530 579 B1**
(45) Date of publication and mention of the grant of the patent: **09.05.2012**
(21) Application number: 02794932.0
(22) Date of filing: 21.08.2002
(51) Int. Cl.: C07K 16/10

(54) **RECOMBINANT ANTIBODIES, AND COMPOSITIONS AND METHODS FOR MAKING AND USING THE SAME**
REKOMBINANTE ANTIKÖRPER SOWIE ZUSAMMENSETZUNGEN UND VERFAHREN ZU DEREN HERSTELLUNG UND VERWENDUNG
ANTICORPS DE RECOMBINAISON, COMPOSITIONS ET TECHNIQUES DE FABRICATION ET D'UTILISATION DE CES ANTICORPS

(30) Priority: 21.08.2001 US 314023 P
(43) Date of publication of application: 18.05.2005
(73) Proprietor: THOMAS JEFFERSON UNIVERSITY, Philadelphia, PA 19107-6799 (US)
(72) Inventor: HOOPER, Douglas, C., Medford, NJ 08055 (US); DIETZSCHOLD, Bernhard, Newtown Square, PA 19073 (US)
(74) Representative: Stark, Gordon Drummond
(86) International application number: PCT/US2002/026584
(87) International publication number: WO 2003/016501

(56) References cited:
- WO-A-01/88132
- CHAMPION J M ET AL: "The development of monoclonal human rabies virus-neutralizing antibodies as a substitute for pooled human immune globulin in the prophylactic treatment of rabies virus exposure" JOURNAL OF IMMUNOLOGICAL METHODS, ELSEVIER SCIENCE PUBLISHERS B.V.,AMSTERDAM, NL, vol. 235, no. 1-2, February 2000 (2000-02), pages 81-90, XP004188234 ISSN: 0022-1759
- CHEUNG S C ET AL: "A RECOMBINANT HUMAN FAB EXPRESSED IN ESCHERICHIA COLI NEUTRALIZES RABIES VIRUS" JOURNAL OF VIROLOGY, THE AMERICAN SOCIETY FOR MICROBIOLOGY, US, vol. 66, no. 11, November 1992 (1992-11), pages 6714-6720, XP001031105 ISSN: 0022-538X
- RANDO R F ET AL: "PRODUCTION OF HUMAN MONOCLONAL ANTIBODIES AGAINST RABIES VIRUS" CURRENT TOPICS IN MICROBIOLOGY AND IMMUNOLOGY, SPRINGER, BERLIN,, DE, vol. 187, 1994, pages 195-205, XP001030996 ISSN: 0070-217X
- IKEMATSU H ET AL: "Clonal analysis of a human antibody response. II. Sequences of the VH genes of human IgM, IgG, IgA to rabies virus reveal preferential utilization of VHIII segments and somatic hypermutation" JOURNAL OF IMMUNOLOGY, THE WILLIAMS AND WILKINS CO. BALTIMORE, US, vol. 150, no. 4, 15 February 1993 (1993-02-15), pages 1325-1337, XP002197277 ISSN: 0022-1767
- CRUSE M J: 'Illustrated Dictionary of Immunology', 1995, CRC PRESS page 143, XP002975947

### SEQUENCE LISTING

<110> Hooper, Douglas
   Dietzschold, Bernhard
<210> 1
   <211> 1430
   <212> DNA
   <213> Homo sapien
<400> 1
   accatggagt ttgggctgag ctggcttttt cttgtggcta ttttaaaagg tgtccagtgt      60
   gaggtgcagc tgttggagtc tgggggaggc ttggtacagc ctggggggtc cctgagactc     120
   tcctgtgcag cctctggatt cacctttagc aactatgcca tgagctgggt ccgccaggct     180
   ccagggaagg ggctggagtg ggtctcagct attagtgcta gtggtcatag cacatatttg     240
   gcagactccg tgaagggccg gttcaccatc tccagagaca attccaagaa cacgctgtat     300
   ctgcaaatga acagcctgag agccgaggac acggccgtat attactgtgc gaaagatcga     360
   gaggttacta tgatagttgt acttaatgga ggctttgact actggggcca gggaacccgg     420
   gtcaccgtct cctccgcctc caccaagggc ccatcggtct tccccctggc accctcctcc     480
   aagagcacct ctgggggcac agcggccctg ggctgcctgg tcaaggacta cttccccgaa     540
   ccggtgacgg tgtcgtggaa ctcaggcgcc ctgaccagcg gcgtgcacac cttcccggct     600
   gtcctacagt cctcaggact ctactccctc agcagcgtgg tgaccgtgcc ctccagcagc     660
   ttgggcaccc agacctacat ctgcaacgtg aatcacaagc ccagcaacac caaggtggac     720
   aagagagttg agcccaaatc ttgtgacaaa actcacacat gcccaccgtg cccagcacct     780
   gaactcctgg ggggaccgtc agtcttcctc ttccccccaa aacccaagga caccctcatg     840
   atctcccgga cccctgaggt cacatgcgtg gtggtggacg tgagccacga agaccctgag     900
   gtcaagttca actggtacgt ggacggcgtg gaggtgcata atgccaagac aaagccgcgg     960
   gaggagcagt acaacagcac gtaccgtgtg gtcagcgtcc tcaccgtcct gcaccaggac    1020
   tggctgaatg gcaaggagta caagtgcaag gtctccaaca aagccctccc agcccccatc    1080
   gagaaaacca tctccaaagc caaagggcag ccccgagaac cacaggtgta caccctgccc    1140
   ccatcccggg aggagatgac caagaaccag gtcagcctga cctgcctggt caaaggcttc    1200
   tatcccagcg acatcgccgt ggagtgggag agcaatgggc agccggagaa caactacaag    1260
   accacgcctc ccgtgctgga ctccgacggc tccttcttcc tctatagcaa gctcaccgtg    1320
   gacaagagca ggtggcagca ggggaacgtc ttctcatgct ccgtgatgca tgaggctctg    1380
   cacaaccact acacgcagaa gagcctctcc ctgtccccgg gtaaatgagt               1430
<210> 2
   <211> 708
   <212> DNA
   <213> Homo sapien
<400> 2
   agcatggaag ccccagctca gcttctcttc ctcctgctac tctggctccc agataccacc      60
   ggagaaattg tgttgacaca gtctccagcc accctgtctt tgtctccagg ggaaagagcc     120
   accctcgcct gcagggccag tcagactgct agcaggtact tagcctggta ccaacagaaa     180
   cctggccagg ctcccagact cctcatctat gatacatcca acagggccac tggcatccca     240
   gccaggttca gtggcagtgg gtctgggaca gacttcactc tctccatcag cagcctggag     300
   cctgaagatt ttgcagttta ttactgtcag cagcgtttca actggccgtg gacgttcggc     360
   caagggacca aggtggaatt caaacgaact gtggctgcac catctgtctt catcttcccg     420
   ccatctgatg agcagttgaa atctggaact gcctctgttg tgtgcctgct gaataacttc     480
   tatcccagag aggccaaagt acagtggaag gtggataacg ccctccaatc gggtaactcc     540
   caggagagtg tcacagagca ggacagcaag gacagcacct acagcctcag cagcaccctg     600
   acgctgagca aagcagacta cgagaaacac aaagtctacg cctgcgaagt cacccatcag     660
   ggcctgagct cgcccgtcac aaagagcttc aacaggggag agtgttag                  708
<210> 3
   <211> 474
   <212> PRT
   <213> Homo sapien
<400> 3
<210> 4
   <211> 234
   <212> PRT
   <213> Homo sapien
<400> 4
<210> 5
   <211> 20
   <212> DNA
   <213> Homo sapien
<400> 5
   accatggagt ttgggctgag 20
<210> 6
   <211> 20
   <212> DNA
   <213> Homo sapien
<400> 6
   actcatttac ccggggacag 20
<210> 7
   <211> 20
   <212> DNA
   <213> Homo sapien
<400> 7
   agcatggaag ccccagctca 20
<210> 8
   <211> 21
   <212> DNA
   <213> Homo sapien
<400> 8 21
   ctctaacact ctcccctgtt g 21

## Description

### FIELD OF THE INVENTION

The present invention relates to recombinant antibodies including the nucleic acid and amino acid sequence of human monoclonal rabies virus-neutralizing antibodies.

### BACKGROUND OF THE INVENTION

Rabies is an acute, neurological disease caused by infection of the central nervous system with rabies virus, a member of the *Lyssavirus* genus of the family *Rhabdoviridae.* Of great historical significance due to its antiquity and the horrific nature of the disease, rabies virus continues to be an important threat of human and veterinary infection because of extensive reservoirs in diverse species of wildlife. Throughout much of the world, distinct variants of rabies virus are endemic in particular terrestrial animal species, with relatively little in common between them. While several islands, including the United Kingdom, Australia, Japan, and numerous islands are free of terrestrial rabies, rabies and rabies-related viruses associated with bats have recently been identified in the UK and Australia.

Rabies virus is characteristically bullet-shaped, enveloped particle of, on average, 75 by 180 nanometers. The virion consists of a single-stranded negative sense RNA genome and five structural proteins: the nucleoprotein (N) molecules, the phospho-protein (NS), the polymerase (L), the matrix protein (M) and the viral glycoprotein (G).

The N and G proteins both bear antigenic determinants which enable serotypic characterization of diverse rabies virus strains. N determinants are highly conserved between different virus isolates and are therefore very useful targets for the immunohistological detection of rabies virus infection using specific antibodies. On the other hand, antigenic determinants carried on the G-protein vary substantially among the rabies virus strains. Virus-neutralizing antibodies raised by vaccination with inactivated virus are directed against G. While it is clear that T cell responses to G, N, and NS, participate in immune responses to the virus under experimental conditions, assessment of immunity to rabies virus is generally limited to serology, particularly with respect to virus-neutralizing antibodies.

In areas of the world where human rabies is still common, the dog is the major reservoir of the viruses that infect man. Where canine rabies has largely been eliminated by vaccination, foxes, coyotes, skunks, raccoons, bats, and a variety of other mammals harbor variants of the virus. In many areas, wildlife reservoirs of virus continue to expand Moreover, rabies virus can be transmitted from a reservoir species to humans or other end stage hosts by animals not normally associated with rabies, such as cats, rabbits, etc.

Almost invariably fatal once clinical symptoms appear, rabies can be averted by prompt treatment of an infected individual with a combination of passive and active immunization. Passive immunization consists of the administration of pre-formed rabies virus neutralizing antibodies obtained from pooled serum of rabies immune individuals (Human rabies-immune globulin; HRIG) or hyper-immunized horses (Equine rabies-immune globulin; ERIG). Both types of reagent present certain risks to recipients including variable antigen specificity, and thus potency, for different rabies virus isolates.

HRIG is prepared from pooled human sera, therefore there is the possibility that HRIG preparations could be contaminated with known or unknown human pathogens. On the other hand, as a preparation of foreign antigen, ERIG has been associated with severe anaphylactic reactions. Mouse monoclonals specific for rabies virus have been contemplated for use in post-exposure prophylaxis but, like ERIG, are antigenically foreign to humans. This may result in their rapid clearance from the human system, as well as the potential to cause an anaphylactic reaction.

The use of human monoclonal antibodies is limited since human hybridoma cell lines are hard to prepare, generally unstable, and do not produce monoclonal antibodies of appropriate specificity in sufficient quantities and at reasonable costs. Production costs of monoclonal antibodies make it desirable to find more economic alternatives to obtaining monoclonal antibodies from hybridomas.

It is well established that both the Fab and Fab2 regions, which comprise the variable and hinge regions of the heavy and light chains, do not protect against rabies virus infection. The *in vivo* efficacy of the antibody relies on the entire sequence, that is only particular antibodies exhibit anti-rabies activity. It is the constant region of the antibody that is responsible for immunoreactivity. Thus, it is particular attributes of the constant region(s) that are required to protect against the rabies virus. Variable regions spliced to a constant region of another antibody, that is an antibody that is not naturally made against the rabies virus, are ineffective.

Champion J. M. et al., Journal of Immunological Methods 235: 81-90, 2000 describes a series of human rabies virus-specific monoclonal antibodies. Cheung S. C. et al., J. Virol. 66: 6714-6720, 1992 describes a method for preparing the Fab domain of rabies-virus neutralizing antibody MAb-57. Hanlon C. A. et al., Vaccine 19: 3834-3842, 2001 describes rabies virus-neutralizing human monoclonal antibodies.

There is a need for recombinant antibodies useful in the diagnosis, prevention and treatment of rabies infection, and pharmaceutical compositions that comprise and methods that use the same. There is a need for compositions and methods for producing such recombinant antibodies.

There is a need for recombinant antibodies useful in the diagnosis, prevention and treatment of infection of pathogens that target neuronal tissue, and pharmaceutical compositions that comprise and methods that use the same. There is a need for compositions and methods for producing recombinant antibodies.

To provide a better reagent, human monoclonal antibodies have been made by fusion of Epstein-Barr Virus (EBV)-transformed, rabies virus-specific human B cells with mouse-human heterohybrid donors. cDNA clones encoding the antibody heavy and light chains from these cells were constructed such that the antibodies were expressed in heterologous expression systems. These constructs allow rabies neutralizing human antibodies of defined specificity to be produced in a controlled system, purified away from possible deleterious contaminants. The present invention relates to these monoclonal rabies virus neutralizing human antibodies, the nucleic acid sequences of their heavy and light chains and the amino acid sequences of the encoded proteins. Also disclosed are methods of using the monoclonal antibodies as a therapeutically effective post-exposure prophylactic treatment of individuals exposed to rabies virus.

### SUMMARY OF THE INVENTION

The present invention provides recombinant antibodies, and compositions for and methods of producing such antibodies. According to some aspects of the invention, the present invention provides recombinant anti-rabies antibodies, and compositions for and methods of producing such antibodies. According to some aspects of the invention, the present invention provides recombinant antibodies with a specific constant region that makes them particularly effective in combating pathogens which attack the neural system.

The present invention further relates to isolated DNA sequences as defined in claims 1 and 2, to recombinant vectors comprising such sequences as defined in claim 3, to host cells comprising such vectors as defined in claim 4 and methods of producing recombinant antibodies using such host cells as defined in claim 5.

Also disclosed is the use of recombinant antibodies in the diagnosis, prevention and treatment of pathogen infections of neuronal tissue, particularly rabies.

The present invention provides isolated nucleic acid molecules as defined in claims 1 and 2 having a heavy chain and a light chain nucleic acid sequence encoding a heavy chain and a light chain amino acid sequence. The heavy chain and light chain amino acid sequences are that of a monoclonal rabies virus neutralizing antibody that specifically binds to a rabies virus protein.

According to one aspect of the present invention there is provided a recombinant antibody as defined in claim 6.

Described herein is an isolated human monoclonal rabies virus neutralizing antibody that is encoded in cDNA clones encoding the antibody heavy and light chains expressed in heterologous expression systems and purified away from deleterious contaminants.

Also disclosed is a fused gene encoding a chimeric immunoglobulin light chain. The chimeric light chain contains a first DNA sequence encoding an immunoglobulin light chain variable region of a monoclonal rabies virus neutralizing antibody produced by a heterohybridoma cell line; and a second DNA sequence encoding a human light chain constant region. Also disclosed is an expression vector to express this fused gene and a host cell for the expression vector.

Also described herein is a fused gene encoding a chimeric immunoglobulin heavy chain. The chimeric heavy chain contains a first DNA sequence encoding an immunoglobulin heavy chain variable region of a monoclonal rabies virus neutralizing antibody produced by a heterohybridoma cell line; and a second DNA sequence encoding a human heavy chain constant region. Described herein is an expression vector to express this fused gene and a host cell for the expression vector.

According to a further aspect of the present invention there is provided a recombinant antibody as defined in claim 7. Described herein is an isolated monoclonal rabies virus neutralizing antibody derived from the fused gene encoding a chimeric immunoglobulin light chain and the fused gene encoding a chimeric immunoglobulin heavy chain.

According to further aspects of the present invention there are provided antibody fragments as defined in claims 8 and 9.

According to further aspects of the present invention there is provided an antibody or antibody fragment for use as a medicament, particularly for use in treating an individual exposed to a rabies virus, as defined in claims 10 to 13.

Described herein is a method of treating an individual exposed to a rabies virus by administering to the individual a therapeutically effective amount of a human monoclonal rabies virus neutralizing antibody that is encoded in cDNA clones encoding the antibody heavy and light chains expressed in heterologous expression systems and purified away from deleterious contaminants, thereby preventing the spread of rabies virus to the central nervous system.

### DESCRIPTION OF THE INVENTION

The present invention provides monoclonal antibodies that bind specifically to the glycoprotein of various rabies virus strains. Post-exposure treatment with monoclonal antibody, or a mixture of a variety of monoclonal antibodies, will neutralize the rabies virus at the site of entry and prevent the virus from spreading to the central nervous system (CNS). Thus, for transdermal or mucosal exposure to rabies virus, rabies specific-monoclonal antibodies are instilled into the bite site, as well as administered systemically. Since viral replication is restricted almost exclusively to neuronal cells, neutralization and clearance of the virus by the monoclonal antibodies of the present invention prior to entry into the CNS is an effective post-exposure prophylactic.

Described herein are sequences of monoclonal antibodies against rabies virus. While most of the variable region of MAb 57 is well known (Cheung et al., J. Virol. 66:6714-6720, 1992), the constant region is not. The entire monoclonal antibody, both constant and variable regions, has been cloned and sequenced. Described herein is the novel nucleotide sequence of MAb 57 constant region, nucleotides 476-1431, which includes constant domain 1 (CH1) and the hinge region. This sequence may be used in recombinant antibodies including anti-rabies antibodies or recombinant antibodies directed at other pathogens which attack neuronal tissue, such as encephalitis or herpes.

The invention relates to the recombinant antibodies, to the cloned genes that encode them, to the vectors which incorporate cloned genes, and to host cells that include the vectors. The invention also provides methods of making and using the recombinant antibodies.

Described herein are recombinant antibodies derived from MAb 57. MAb 57 derived from hybridomas are IgG2 antibodies; recombinant antibodies derived from MAb 57 are IgG1 antibodies. Described herein are recombinant antibodies derived from MAb 57, to the clones genes that encode them, to the vectors which incorporate cloned genes and host cells that include the vectors. Also described herein are methods of making and using the recombinant antibodies.

Also described herein is the entire sequence of the heavy and light chains of the anti-rabies monoclonal antibody MAb JA. Described herein are recombinant antibodies derived from MAb JA, to the clones genes that encode them, to the vectors which incorporate cloned genes and host cells that include the vectors.

Also described herein are methods of making and using the recombinant antibodies.

The present invention also provides the entire sequence of the heavy and light chains of the anti-rabies monoclonal antibody MAb JB.1. The invention relates to the recombinant antibodies derived from MAb JB.1, to the clones genes that encode them, to the vectors which incorporate cloned genes and host cells that include the vectors. Also described are methods of making and using the recombinant antibodies.

Described herein is a recombinant antibody being a single-chain antibody wherein the heavy chain variable domain and the light chain variable domain are linked by way of a spacer group, preferably a peptide. Most preferred is a single-chain antibody wherein the heavy chain variable domain is located at the N-terminus of the recombinant antibody. The single-chain recombinant antibody may further comprise an effector molecule and/or signal sequences facilitating the processing of the antibody by the host cell in which it is prepared.

The recombinant antibodies of the invention can be used to identify rabies virus such as by immunofluorescent staining of infected cells, by immunoblotting either directly or by way of immunoprecipitation and protein blotting of the immunocomplexes, or by another immunoassay such as a binding, crossinhibition or competition radio- or enzyme immunoassay.

Described herein is a method of manufacture of the recombinant antibodies of the invention. The recombinant antibodies of the invention can be prepared by recombinant DNA techniques comprising culturing a transformed host under conditions which allow expression thereof and isolating said antibody.

Also described herein is a process for the production of a recombinant antibody comprising culturing a host which has been transformed with a hybrid vector comprising an expression cassette comprising a promoter and a DNA coding for said recombinant antibody which DNA is controlled by said promoter, and isolating said recombinant antibody.

*In vitro* production provides relatively pure antibody preparations and allows scale-up to give large amounts of the desired antibodies. Techniques for bacterial cell, yeast or mammalian cell cultivation are known in the art and include homogeneous suspension culture, e.g. in an airlift reactor or in a continuous stirrer reactor, or immobilized or entrapped cell culture, e.g. in hollow fibres, microcapsules, on agarose microbeads or ceramic cartridges.

Degenerated sequences are degenerated within the meaning of the genetic code in that an unlimited number of nucleotides are replaced by other nucleotides without resulting in a change of the amino acid sequence originally encoded. Such degenerated sequences may be useful due to their different restriction sites and/or frequency of particular codons which are preferred by the specific host, particularly E. coli, to obtain an optimal expression of the recombinant antibody.

Described herein is a recombinant DNA which is a hybrid vector comprising an insert coding for the recombinant antibody described hereinbefore, and, optionally an origin of replication or an autonomously replicating sequence, one or more dominant marker sequences, expression control sequences, signal sequences and additional restriction sites.

Vectors typically perform two functions in collaboration with compatible host cells. One function is to facilitate the cloning of the nucleic acid that encodes the immunoglobulin domains, i.e. to produce usable quantities of the nucleic acid (cloning vectors). The other function is to provide for replication and expression of the recombinant gene constructs in a suitable host, either by maintenance as an extrachromosomal element or by integration into the host chromosome (expression vectors). A cloning vector comprises the recombinant gene constructs as described above, an origin of replication or an autonomously replicating sequence, dominant marker sequences and, optionally, signal sequences and additional restriction sites. An expression vector additionally comprises expression control sequences essential for the transcription and translation of the recombinant genes.

An origin of replication or an autonomously replicating sequence is provided either by construction of the vector to include an exogeneous origin such as derived from Simian virus 40 (SV 40) or another viral source, or by the host cell chromosomal mechanisms.

The markers allow for selection of host cells which contain the vector. Selection markers include genes which confer resistance to heavy metals such as copper or to antibiotics such as geneticin (G-418) or hygromycin, or genes which complement a genetic lesion of the host cell such as the absence of thymidin kinase, hypoxanthine phosphoryl transferase, dihydrofolate reductase or the like.

Signal sequences may be, for example, presequences or secretory leaders directing the secretion of the recombinant antibody, splice signals, or the like. Examples for signal sequences directing the secretion of the recombinant antibody are sequences derived from the ompA gene, the pelB (pectate lyase) gene or the phoA gene.

As expression control sequences, the vector DNA comprises a promoter, sequences necessary for the initiation and termination of transcription and for stabilizing the mRNA and, optionally, enhancers and further regulatory sequences.

A wide variety of promoting sequences may be employed, depending on the nature of the host cell. Promoters that are strong and at the same time well regulated are the most useful. Sequences for the initiation of translation are for example Shine-Dalgarno sequences. Sequences necessary for the initiation and termination of transcription and for stabilizing the mRNA are commonly available from the noncoding 5'-regions and 3'-regions, respectively, of viral or eukaryotic cDNAs, e.g. from the expression host. Enhancers are transcription-stimulating DNA sequences of viral origin, e.g. derived from Simian virus, polyoma virus, bovine papilloma virus or Moloney sarcoma virus, or of genomic, especially murine, origin.

The various DNA segments of the vector DNA are operationally linked, i.e. they are contiguous and placed into a functional relationship with each other. Examples of vectors which are suitable for replication and expression in an E. coli strain are bacteriophages, for example derivatives of .lambda. bacteriophages, or plasmids, such as, in particular, the plasmid ColE1 and its derivatives, for example pMB9, pSF2124, pBR317 or pBR322 and plasmids derived from pBR322, such as pUC9, pUCK0, pHRi148 and pLc24. Suitable vectors contain a complete replicon, a marker gene, recognition sequences for restriction endonucleases, so that the foreign DNA and, if appropriate, the expression control sequence can be inserted at these sites, and optionally signal sequences and enhancers.

Microbial promoters are, for example, the strong leftward promoter P_{L} of bacteriophage λ. which is controlled by a temperature sensitive repressor. Also suitable are E. coli promoters such as the lac (lactose) promoter regulated by the lac repressor and induced by isopropyl-.beta.-D-thiogalactoside, the trp (tryptophan) promoter regulated by the trp repressor and induced e.g. by tryptophan starvation, and the tac (hybrid trp-lac promoter) regulated by the lac repressor.

Vectors which are suitable for replication and expression in yeast contain a yeast replication start and a selective genetic marker for yeast. One group of such vectors includes so-called ars sequences (autonomous replication sequences) as origin of replication. These vectors are retained extrachromosomally within the yeast cell after the transformation and are replicated autonomously. Furthermore, vectors which contain all or part of the 2µm plasmid DNA from Saccharomyces cerevisiae can be used. Such vectors will get integrated by recombination into 2µm plasmids already existing within the cell, or replicate autonomously. 2µm sequences are particularly suitable when high transformation frequency and high copy numbers are to be achieved.

Expression control sequences which are suitable for expression in yeast are, for example, those of highly expressed yeast genes. Thus, the promoters for the TRP1 gene, the ADHI or ADHII gene, acid phosphatase (PHO3 or PHO5) gene, isocytochrome gene or a promoter involved with the glycolytic pathway, such as the promoter of the enolase, glyceraldehyde-3-phosphate kinase (PGK), hexokinase, pyruvate decarboxylase, phosphofructokinase, glucose-6-phosphate isomerase, 3-phosphoglycerate mutase, pyruvate kinase, triosephosphate isomerase, phosphoglucose isomerase and glucokinase genes, can be used.

Vectors suitable for replication and expression in mammalian cells are preferably provided with promoting sequences derived from DNA of viral origin, e.g. from Simian virus 40 (SV40), Rous sarcoma virus (RSV), adenovirus 2, bovine papilloma virus (BPV), papova-virus BK mutant (BKV), or mouse or human cytomegalovirus (CMV). Alternatively, the vectors may comprise promoters from mammalian expression products, such as actin, collagen, myosin etc., or the native promoter and control sequences which are normally associated with the desired gene sequence, i.e. the immunoglobulin H-chain or L-chain promoter.

Some preferred vectors are suitable for both procaryotic and eucaryotic hosts and are based on viral replication systems. Particularly preferred are vectors comprising Simian virus promoters, e.g. pSVgpt or pSVneo, further comprising an enhancer, e.g. an enhancer normally associated with the immunoglobulin gene sequences, in particular the mouse Ig H- or L-chain enhancer.

The recombinant DNA coding for a recombinant antibody of the invention can be prepared, for example, by culturing a transformed host cell and optionally isolating the prepared DNA.

Also described herein are host cells transformed with the recombinant DNAs described above, namely host cells which are transformed with a DNA encoding the heavy chain and/or a DNA encoding the light chain of the desired recombinant antibody, in particular host cells transformed with a DNA encoding the single-chain recombinant antibody.

Described herein is a host cell which has been transformed with a hybrid vector comprising an expression cassette comprising a promoter and a DNA coding for a recombinant antibody.

Furthermore, described herein is a host cell which has been transformed with a hybrid vector comprising an expression cassette comprising a promoter operably linked to a first DNA sequence encoding a signal peptide linked in the proper reading frame to a second DNA sequence encoding a recombinant antibody.

Examples of suitable hosts are microorganisms which are devoid of or poor in restriction enzymes or modification enzymes, such as bacteria, in particular strains of Escherichia coli, for example E. coli X1776, E. coli Y1090, E. coli HB 101, E. coli W3110, E. coli HB 101/LM1035, E. coli JA 221, E. coli DH5. alpha., E. coli K12, or E. coli CC118 strain, Bacillus subtilis, Bacillus stearothermophilus, Pseudomonas, Haemophilus, Streptococcus and others, and yeasts, for example Saccharomyces cerevisiae such as S. cerevisiae GRF 18. Further suitable host cells are cells of higher organisms, in particular established continuous human or animal cell lines, e.g. human embryonic lung fibroblasts L132, human malignant melanoma Bowes cells, HeLa cells, SV40 virus transformed kidney cells of African green monkey COS-7 or Chinese hamster ovary (CHO) cells, or cells of lymphoid origin, such as lymphoma, myeloma, hybridoma, trioma or quadroma cells, for example PAI, Sp2/0 or X63-Ag8. 653 cells.

Described herein are processes for the preparation of transformed host cells wherein suitable recipient host cells as described hereinbefore are transformed with a hybrid vector according to the invention, and the transformed cells are selected. Transformation of microorganisms is carried out as described in the literature, for example for S. cerevisiae (A. Hinnen et al., Proc. Natl. Acad. Sci. USA 75: 1929, 1978), for B. subtilis (Anagnostopoulos et al., J. Bacteriol. 81: 741, 1961), and for E. coli (M. Mandel et al., J. Mol. Biol. 53: 159, 1970).

Accordingly, the transformation procedure ofE. coli cells includes, for example, Ca²⁺ pretreatment of the cells so as to allow DNA uptake, and incubation with the hybrid vector. The subsequent selection of the transformed cells can be achieved, for example, by transferring the cells to a selective growth medium which allows separation of the transformed cells from the parent cells dependent on the nature of the marker sequence of the vector DNA. Preferably, a growth medium is used which does not allow growth of cells which do not contain the vector. The transformation of yeast comprises, for example, steps of enzymatic removal of the yeast cell wall by means of glucosidases, treatment of the obtained spheroplasts with the vector in the presence of polyethylene glycol and Ca²⁺ ions, and regeneration of the cell wall by embedding the spheroplasts into agar. Preferably, the regeneration agar is prepared in a way to allow regeneration and selection of the transformed cells as described above at the same time.

Transformation of cells of higher eucaryotic origin, such as mammalian cell lines, is preferably achieved by transfection. Transfection is carried out by conventional techniques, such as calcium phosphate precipitation, microinjection, protoplast fusion, electroporation, i.e. introduction of DNA by a short electrical pulse which transiently increases the permeability of the cell membrane, or in the presence of helper compounds such as diethylaminoethyldextran, dimethyl sulfoxide, glycerol or polyethylene glycol, and the like. After the transfection procedure, transfected cells are identified and selected, for example, by cultivation in a selective medium chosen depending on the nature of the selection marker, for example standard culture media such as Dulbecco's modified Eagle medium (DMEM), minimum essential medium, RPMI 1640 medium and the like, containing e.g. the corresponding antibiotic.

The recombinant antibodies according to the invention can be used for the qualitative and quantitative determination of the presence of rabies virus. In general, the recombinant antibodies according to the invention can be used in any of the known immunoassays which rely on the binding interaction between the antibodies and rabies antigens. Examples of such assays are radio-, enzyme, fluorescence, chemiluminescence, immunoprecipitation, latex agglutination, and hemagglutination immunoassays, and, in particular, immunostaining methods.

The antibodies according to the invention can be used as such or in the form of enzyme-conjugated derivatives in an enzyme immunoassay. Any of the known modifications of an enzyme immunoassay can be used, for example soluble phase (homogeneous) enzyme immunoassay, solid phase (heterogeneous) enzyme immunoassay, single enzyme immunoassay or double (sandwich) enzyme immunoassay with direct or indirect (competitive) determination of the presence of rabies virus.

An example of such an enzyme immunoassay is a sandwich enzyme immunoassay in which a suitable carrier, for example the plastic surface of a microtiter plate or of a test tube, e.g. of polystyrene, polypropylene or p6lyvinylchloride, glass or plastic beads, filter paper, dextran etc. cellulose acetate or nitrocellulose sheets, magnetic particles or the like, is coated with a monoclonal antibody of the invention by simple adsorption or optionally after activation of the carrier, for example with glutaraldehyde or cyanogen bromide. Then test solutions containing the rabies virus and finally recombinant antibodies of the invention comprising a detectable enzyme, e.g. alkaline phosphatase, are added. The amount of the rabies virus in the test solution is directly proportional to the amount of bound recombinant antibody and is determined by adding an enzyme substrate solution. The enzyme substrate reaction results, for example, in a color change which can be observed by eye or with optical measuring devices.

The antibodies according to the invention can be used as such or in the form of radioactively labelled derivatives in a radioimmunoassay (RIA). As described above for enzyme immunoassays, any of the known modifications of a radioimmunoassay can be used.

The tests are carried out in an analogous manner to the enzyme immunoassays described above using a radioactive label, e.g. ^{I2S}I, instead of an enzyme label. The amount of immune complex formed which corresponds to the amount of rabies virus present in the test solutions is determined by measuring the radioactivity of the immune complex.

For immunostaining cryosections of cryopreserved biopsy material or paraffin embedded tissue sections are treated with a solution containing a recombinant antibody of the invention comprising a detectable enzyme. Bound recombinant antibody is detected by treatment with a suitable enzyme substrate, preferably an enzyme substrate which leads to a solid deposit (stain) at the site of the recombinant antibody of the invention. In place of recombinant antibodies comprising an enzyme, a recombinant antibody comprising streptavidin and a solution of a biotin-enzyme-conjugate may be used, which leads to higher enzyme concentration at the site of the antibody and hence increased sensitivity of the immunostaining method. The solid deposit of the enzyme substrate is detected by inspection with a microscope, for example with a fluorescence microscope, or by scanning the optical density at the wavelength of the stain.

The use of recombinant antibodies as described hereinbefore for the determination of rabies virus also includes other immunoassays known per se, for example immunofluorescence assays, latex agglutination with antibody-coated or antigen coated latex particles, hemagglutination with antibody-coated or antigen-coated red blood corpuscles, evanescent light assays using an antibody-coated optical fibre and other direct-acting immunosensors which convert the binding event into an electrical or optical signal, or the like.

Also described herein are test kits for the qualitative and quantitative determination of presence of rabies virus comprising recombinant antibodies of the invention and, optionally, adjuncts, positive and/or negative controls, buffers, instructions and descriptions of exemplary results.

Furthermore, the recombinant antibodies of the invention, are useful for the prevention of rabies infection in patients suspected of possible exposure to rabies virus or the treatment of patients who have been infected with rabies.

Described herein are pharmaceutical compositions comprising a therapeutically effective amount of a recombinant antibody according to the invention and a pharmaceutically acceptable carrier. Preferred are pharmaceutical compositions for parenteral application. Compositions for intramuscular, subcutaneous or intravenous application are e.g. isotonic aqueous solutions or suspensions, optionally prepared shortly before use from lyophilized or concentrated preparations. Suspensions in oil contain as oily component the vegetable, synthetic or semi-synthetic oils customary for injection purposes. The pharmaceutical compositions may be sterilized and contain adjuncts, e.g. for conserving, stabilizing, wetting, emulsifying or solubilizing the ingredients, salts for the regulation of the osmotic pressure, buffer and/or compounds regulating the viscosity, e.g. sodium carboxycellulose, carboxymethylcellulose, sodium carboxymethylcellulose, dextran, polyvinylpyrrolidine or gelatine.

The pharmaceutical compositions contain from approximately 0.01% to approximately 50% of active ingredients. They may be in dosage unit form, such as ready-to-use ampoules or vials, or also in lyophylized solid form.

In general, the prophylactically and therapeutically effective doses for mammals is between approximately 5 and 250 pg of a recombinant antibody of the invention per kg body weight depending on the type of antibody, the status of the patient and the mode of application. The specific mode of administration and the appropriate dosage will be selected by the attending physician taking into account the particulars of the patient, the state of the disease, the type of tumor treated, and the like. The pharmaceutical compositions are prepared by methods known in the art, e.g. by conventional mixing, dissolving, confectioning or lyophilizing processes. Pharmaceutical compositions for injection are processed, filled into ampoules or vials, and sealed under aseptic conditions according to methods known in the art.

Described herein are antibody cocktails in which one or more antibodies are combined. The cocktails may contain two or more antibodies of the present invention.

### EXAMPLE

### Example 1

### Cells

The human B cells used for hybridization were obtained from the peripheral blood of 5 donors between 7 and 21 days after the third dose of a primary rabies vaccination and 5 rabies-immune donors 10 to 21 days following administration of booster vaccine. In all cases the vaccine employed was Rabivac™ human diploid cell vaccine (virus strain Pitman Moore 1503-3M, Behringwerke, Marburg, FRG). All of the donors were negative in tests for HIV and hepatitis B. The mouse-human hybrid heteromyeloma SHM-D33 cells utilized as hybridoma fusion partners (Teng, N.N. et al., Proc. Natl. Acad. Sci. USA 80, 7308, 1983) and B95-8 Epstein-Barr Virus (EBV)-transformed marmoset leukocytes used as a source ofEBV (Henderson et al., J. Exp.:Med. Vol 76, p. 152, 1977) were obtained from ATCC (Rockville, MD).

### Rabies viruses

To assess the capacity of antibody preparations to neutralize a variety of rabies virus strains, a number of antigenically distinct fixed, laboratory strains, as well as two representative street rabies viruses, were used. Evelyn-Rokitnicki-Abelseth (ERA), challenge virus standard, either mouse brain adapted (CVS-24) or cell culture adapted (CVS-11), and Pitman-Moore (PM) fixed strains were obtained from the Thomas Jefferson University virus collection. Silver-haired bat rabies virus (SHBRV), which has been associated with most of the recent rabies cases in the United States of America, and coyote street rabies virus/Mexican dog rabies virus (COSRV), which is a member of the dog rabies viruses, were obtained as described (Morimoto et al., Proc. Natl. Acad. Sci. USA, Vol. 93, p. 5653, 1996). Virus purification and preparation of glycoprotein (G) and nucleoprotein (N) have been described elsewhere (Dietzschold et al., World Health Organization, Geneva, p. 175, 1996).

### EBV-transformation of human PBLs

Peripheral blood mononuclear cells (PBMCs) were isolated from whole blood by density centrifugation on Ficoll-Paque (Amersham Pharmacia Biotech, Piscataway, NJ) as detailed elsewhere (Plebanski et al., Immunology Vol. 75, p. 86, 1992). T cells were then depleted by negative selection using monoclonal anti-CD2 antibody-coated magnetic beads (Dynal Inc., Lake Success NY) and a magnetic particle concentrator (Dynal). CD-2-negative cells, primarily B cells, were collected and immortalized as previously described (Swaminathan, 1992). Briefly, B95-8 cells, cultured to confluency in RPMI₁₆₄₀ (Gibco BRL Life Technologies, Grand Island NY) supplemented with 10% fetal bovine serum (FBS; Gibco), were lysed by freeze-thawing on dry ice to release intracellular EBV. Supernatant containing EBV was clarified by spinning at 1000 RPM for 10 min and by filtration through a 0.45µm filter. Virus was concentrated by centrifugation at 8000 RPM for 2 h at 4°C. 7 X 10⁶ B cells (suspended in 1ml of B95-8 culture media) were incubated at 37°C for 2 h with virus prepared from 25 mls of B95-8 cells. Following infection, the cells were washed twice with culture media, plated in 96 well flat-bottom microtiter plates (Nunc, Fisher Scientific, Pittsburgh, PA) at a concentration of 1 x 10⁴ cells/well, and cultured at 37°C in a humidified atmosphere of 5% CO₂ and 95% air.

### Establishment of mouse-human hetereohybrids

After the EBV-transformed cell lines had been cultured for approximately 4 weeks, supernatant was harvested and tested for the presence of rabies virus-specific antibody in ELISA. Positive wells were transferred first to 1ml and then to 2ml cultures (48 and 24 well plates, Nunc) and the supernatant then assayed in the rapid fluorescent focus inhibition test (RFFIT) for rabies virus neutralizing antibody, as detailed elsewhere (Hooper, ASM Press, WA p. 755, 1997). Cell lines producing neutralizing antibody were hybridized with SHM-D33 cells (ATCC Accession Number CRL1668) as follows. Equal numbers of SHM-D33 and EBV-transformed cells (approximately 5 x 10⁶ each) were added together into a sterile polystyrene round-bottom tube (Falcon, Fisher Scientific) and centrifuged at 1000 RPM for 10 min. Cells were washed twice with serum-free medium and the cell pellet resuspended in 100 µl of medium.

Tubes were warmed in a 37 °C water bath for 1 min and then 0.5 ml of warm (37°C) 50% (wt/vol) polyethylene glycol (Sigma Chemical Co., St. Louis, MO, cat. # P-7181) was added, dropwise over a 45-sec period while gently shaking the tube. The fusion reaction was then stopped by the slow addition of 3 ml of serum-free medium over 30 sec followed by the addition of 9 ml over 30 sec. The tubes were allowed to stand at room temperature for 8 min and then incubated for 2 min in a 37°C water bath. The cells were then centrifuged at 500 g for 3 min and the cell pellet gently resuspended in 30 ml of Iscove's modification of Dulbecco's (IMDM; Gibco) medium containing 10% FBS, as well as 0.04pM aminopterin (Gibco) and 10µM oubain (Sigma) to select against cells which had not hybridized. Cell suspensions were plated in 96 well flat-bottom microtiter plates at a concentration of 1 x 10⁴ cells per well and incubated as described for the lines.

When colonies ofheterohybrid cells had become established (approximately 6 weeks of culture) supernatants were tested for rabies virus-specific antibody production in ELISA and RFFIT. Antibody-producing cells were cloned a minimum of three times by limiting dilution in microtiter plates. Cells were titrated in 96 well round bottom plates in 2-fold dilutions starting from 4 cells per well. Cells from wells containing an average of 0.25 cells or less were expanded for the collection of supernatant and further analysis.

### Analysis of rabies virus-specific antibodies in ELISA

Antibody specificity and isotype was assessed in solid phase ELISA. Plates (PolySorb™, Nunc) were coated at room temperature in a humidified chamber overnight with 5*g/ml rabies ERA virus, glycoprotein, or nucleoprotein diluted in phosphate-buffered saline (PBS). The plates were then blocked with 5%, powdered milk in PBS and washed in PBS containing 0.05% Tween₂₀ (PBS-Tween) prior to the addition of supernatant samples.

Following incubation at room temperature for 2 h, the plates were washed with PBS-Tween to remove unbound primary antibody and various enzyme-conjugated or biotinylated secondary antibodies specific for the various human heavy chain isotypes were added for 1 h at room temperature. Secondary antibody was detected either by the production of a soluble end product in the medium upon addition of the appropriate substrate ( 3,3',5,5'-tetramethylbenzidine (TMB) in phosphate-citrate buffer, or p-nitrophenyl phosphate (PNPP) in 0.1Mglycine bluffer, Sigma) or following the addition of avidin-alkaline phosphatase (30 min at RT) and PNPP substrate. The peroxidase-TMB reaction was stopped bye the addition of 2M H₂SO₄. Absorbance values were read in a microplate spectrophotometer (Biotek, Winooski VT) at 450 nm for the TMB product and at 405 nm for the PNPP reaction.

### RFFIT

Supernatant samples from each transformed cell line were assayed for the presence of rabies virus-neutralizing antibodies using a variation of the rapid fluorescent focus inhibition test (RFFIT) as previously described (Hooper, ASM Press, WA p. 1997). Supernatant samples (50 µl) were diluted in 96 well flat-bottom plates (Nune). Rabies virus dilution known to cause 80-90% infection of the indicator cells were added to each test sample, and the plates incubated at 37°C for 1 h. Negative media and positive rabies-immune serum control samples were included in each assay. After incubation, 30ul of a 1.8 x 10⁶ cells/ml concentration of baby hamster kidney (BHK) cells were added to each well and cultures incubated overnight at 37°C. The plates were then washed once with ice-cold PBS and fixed with ice-cold 90% acetone for 20 min at -20°C. After fixation, acetone was removed and the plates were air dried. To detect infected BHK cells, 40 ul of FTTC anti-rabies nucleoprotein monoclonal globulin (Centocor, Malvern PA) were added to each well for 45 min at 37°C. The plates were then washed three times with distilled water and examined under a fluorescent microscope.

### Purification of antibodies by affinity chromatography

IgG1 antibody was purified using a protein A column (rProtein A Sepharose™ Fast Flow, Amersham Pharmacia Biotech). Briefly, supernatants were clarified by filtration through a 0.45µm membrane and the pH adjusted to 8.0 with 1N NaOH. Supernatant was run through the column at a linear flow rate of approximately 100 cm/hour. After washing in PBS (pH 8), antibody was eluted from the column using a 0.1M citric acid solution and then dialyzed against PBS.

IgG3 antibody was purified using a protein G column (Protein G Sepharose™ Fast Flow, Amersham Pharmacia Biotech). IgG3-containing supernatant was clarified by filtration through a 0.45µm membrane and the pH adjusted to 7.0 with IN NaOH. Supernatant was run through the column at a linear flow rate of approximately 11 cm/hour. After washing with PBS, antibody was eluted from the column using 0.1M glycine buffer, pH 3.0, and then dialyzed against PBS.

IgM antibody was purified using mannan binding protein and a modification of a previously described technique (Nevens et al., J. Chromatogr, Vol. 597, p. 247, 1992). Briefly, supernatant containing IgM was EDTA treated, brought to pH 8.0 with 1M NaOH, filtered and cooled to 4°C. Mannan binding protein-agarose (Sigma) was washed in a column at 4°C with buffer consisting of 0.1M Na**CO₃ /0.5M NaC1, pH 8.3 and then the supernatant was added and incubated on the column for 15 min at 4°C. The column was then washed with several volumes of binding buffer and brought to RT for 1h. The IgM was eluted from the column with binding buffer at RT and dialyzed against PBS.

Protein concentrations of the dialyzed antibody preparations were determined using a protein detection assay (Bio-Rad Laboratories, Hercules CA) as follows. 100µl of sample were added to 5 ml of a 1/5 dilution of dye reagent concentrate and incubated at RT for 10 minutes. Negative PBS control and various bovine serum albumin (BSA) protein standards were included in each assay. After incubation, samples were read in a spectrophotometer at 595nm. Protein concentrations of test samples were calculated with reference to the absorbance of the BSA standards. The purity of all antibody preparations was assessed by electrophoresis in 12.5% polyacrylamide gel under reducing conditions (SDS-PAGE). Purified antibodies showed two major bands on SDS-PAGE corresponding to isolated heavy and light immunoglobulin chains.

### Generation, isolation and sequencing of cDNA clones

Total RNA was isolated from JA hybridoma cell by using RNAzol B (Biotecx Laboratories, Houston). Reverse transcriptase reactions were performed at 42°C for 1 hr with avian myeloblastosis virus reverse transcriptase (Promega) and oligo(dT) primer. A portion of the reverse transcriptase products were subjected to polymerase chain reaction (PCR) amplification using heavy chain specific primers: IgG-HF1 primer (5'-ACCATGGAGTTTGGGCTGAG-3' (SEQ. ID. NO: 5), start codon; underline, accession # Y14737), and IgG-HR2 primer (5'-ACTCATTTACCCGGGGACAG-3' (SEQ. ID. NO: 6), stop codon; underline, accession # Y14737) or light chain specific primers: IgG-LF5 primer (5'-AGCATGGAAGCCCCAGCTCA-3' (SEQ. ID. NO: 7), start codon; underline, accession # M63438), and IgG-LR2 primer (5'-CTCTAACACTCTCCCCTGTTG-3' (SEQ. ID. NO: 8), stop codon, underline accession # M63438). Amplification was carried out for 35 cycles of denaturation at 94°C for 60 seconds, annealing at 50°C for 60 seconds, and polymerization at 72°C for 90 seconds with Taq DNA polymerase (Promega). The PCR products (1.4 kb for heavy chain, 7 kb for light chain) were purified and sequenced by using the AmpliTaq cycle sequencing kit (Perkin-Elmer) with the specific primers. The PCR products were cloned into TA cloning vector, pCR2.1 (Invitrogen). The cloned heavy chain and light chain cDNA was sequenced by using the AmpliTaq cycle sequencing kit (Perkin-Elmer) with the specific primers.

### Monoclonal rabies virus neutralizing antibody coding sequences

Monoclonal antibody cDNA, and sequences complementary thereto, include monoclonal antibody nucleic acids provided by the present invention. A monoclonal antibody cDNA sequence is provided for the heavy chain (SEQ. ID. NO: 1) and the light chain (SEQ. ID. NO:2) of the monoclonal antibody from clone JA, thus lacking any introns. Described herein are single-stranded oligonucleotides for use as primers in PCR that amplify a monoclonal antibody sequence-containing fragment, for example the variable or hypervariable region of the monoclonal antibody. The oligonucleotide having the sequence of a hybridizable portion, at least 8 nucleotides, of a monoclonal antibody gene, and another oligonucleotide having the reverse complement of a downstream sequence in the same strand of the monoclonal antibody gene, such that each oligonucleotide primes synthesis in a direction toward the other. The oligonucleotides are preferably in the range of 10-35 nucleotides in length.

Described herein is the full-length cDNA sequences for the heavy and light chains of the monoclonal antibody of heterohybridoma clone JA (SEQ. ID. NO: 1 and SEQ. ID NO:2, respectively), and the encoded polypeptides of #1-474 amino acids for the heavy chain (SEQ. ID. NO:3) and #1-234 amino acids for the light chain (SEQ. ID. NO:4).

### Functional equivalents of monoclonal rabies virus neutralizing antibodies

Functional equivalents have binding characteristics comparable to those of the antibodies, and include, for example, chimerized and single chain antibodies, as well as fragments thereof. Methods of producing such functional equivalents are disclosed in PCT Application WO 93/21319, European Patent Application No. 239,400; PCT Application WO 89/09622; European Patent Application 338,745; and European Patent Application EP 332,424.

Functional equivalents include polypeptides with amino acid sequences substantially the same as the amino acid sequence of the variable of hypervanable regions of the antibodies of the present invention. "Substantially the same" amino acid sequence is defined herein as a sequence with at least 70%, preferably at least about 80%, and more preferably at least about 90% homology to another amino acid sequence, as determined by the FASTA search method in accordance with Pearson and Lipman, Proc. Natl. Inst. Acad Sci. USA 85, 2444-2448, 1988. Chimerized antibodies have constant regions derived substantially or exclusively from human antibody constant regions and variable regions derived substantially or exclusively from the sequence of the variable region of a monoclonal antibody from each stable heterohybridoma (Champion, J.M., et al., Journal of Immunological Methods, 235 81-90, 2000).

Single chain antibodies or Fv fragments are polypeptides that consist of the variable region of the heavy chain of the antibody linked to the variable region of the light chain, with or without an interconnecting linker. Thus, the Fv comprises the entire antibody combining site.

Functional equivalents further include fragments of antibodies that have the same, or substantially the same, binding characteristics to those of the whole antibody. Such fragments may contain one or both Fab fragments of the F(ab')₂ fragment. The antibody fragments contain all the six complement determining regions of the whole antibody, although fragments containing fewer than all of such regions, such as three, four or five complement determining regions, are also functional. The functional equivalents are members of the IgG immunoglobulin class and subclasses thereof, but may be or may combine any one of the following immunoglobulin classes: IgM, IgA, IgD, or IgE, and subclasses thereof. Heavy chains of various subclasses, such as the IgG subclasses, are responsible for different effector functions and thus, by choosing the desired heavy chain constant region, chimeric antibodies with desired effector function are produced. Preferred constant regions are gamma 1 (IgG1), gamma 3 (IgG3) and gamma 4 (IgG4). The light chain constant region can be of the kappa or lambda type.

The immunoglobulins of the present invention can be monovalent, divalent or polyvalent. Monovalent immunoglobulins are dimers (HL) formed of a chimeric heavy chain associated through disulfide bridges with a chimeric light chain. Divalent immunoglobulins are tetramers (H₂ L₂) formed of two dimers associated through at least one disulfide bridge.

### Standard recombinant DNA techniques

Standard recombinant DNA techniques are described in Sambrook et al., "Molecular Cloning," Second Edition, Cold Spring Harbor Laboratory Press (1987) and by Ausubel et al. (Eds) "Current Protocols in Molecular Biology," Green Publishing Associates/Wiley-Interscience, New York (1990).

Briefly, a suitable source of cells containing nucleic acid molecules that express the desired DNA, such as an antibody or antibody equivalent, is selected. Total RNA is prepared by standard procedures from a suitable source. The total RNA is used to direct cDNA synthesis. Standard methods for isolating RNA and synthesizing cDNA are provided in standard manuals of molecular biology such as, for example, those described above.

The cDNA may be amplified by known methods. For example, the cDNA may be used as a template for amplification by polymerase chain reaction (PCR); see Saiki et al., Science, 239, 487, 1998 or Mullis et al., U.S. Pat. No. 4,683,195. The sequences of the nucleotide primers for the PCR amplification are derived from the known sequence to be amplified. The oligonucleotides are synthesized by methods known in the art. Suitable methods include those described by Caruthers in Science 230, 281-285, 1985.

A mixture of upstream and downstream oligonucleotides are used in the PCR amplification. The conditions are optimized for each particular primer pair of according to standard procedures. The PCR product is analyzed, for example, by electrophoresis for cDNA having the correct size, corresponding to the sequence between the primers.

Alternatively, the coding region may be amplified in two or more overlapping fragments. The overlapping fragments are designed to include a restriction site permitting the assembly of the intact cDNA from the fragments.

In order to isolate the entire protein-coding regions for the heavy and light chains of each monoclonal antibody from each heterohybridoma cell line, for example, the upstream PCR oligonucleotide primer is complementary to the sequence at the 5' end, encompassing the ATG start codon and at least 5-10 nucleotides upstream of the start codon. The downstream PCR oligonucleotide primer is complementary to the sequence at the 3' end of the desired DNA sequence. The desired cDNA encodes the entire portion of the heavy and light chains of each monoclonal antibody, including the stop codon.

The cDNA to be amplified, such as the encoding the antibodies or antibody equivalents, may also be replicated in a wide variety of cloning vectors in a wide variety of host cells. The host cell may be prokaryotic or eukaryotic.

The vector into which the monoclonal antibody cDNA is spliced may comprise segments of chromosomal, non-chromosomal and synthetic DNA sequences. Some suitable prokaryotic cloning vectors include, but are not limited to, plasmids from *E. coli,* such as colE1, pCR1, pBR322, pMB9, pUC, pKSM, and RP4. Prokaryotic vectors also include, but are not limited to, derivatives of phage DNA such as M 13 and other filamentous single-stranded DNA phages.

The vector containing the monoclonal antibody cDNA to be expressed is transfected into a suitable host cell, as described *infra.* The host cell is maintained in an appropriate culture medium, and subjected to conditions under which the cells and the vector replicate.

### Chimeric antibodies

In general, the chimeric antibodies are produced by preparing, for each of the light and heavy chain components of the chimeric immunoglobulin, a fused gene comprising a first DNA segment that encodes at least the functional portion of the human rabies virus specific neutralizing, preferably glycoprotein, human variable region linked (e.g., functionally rearranged variable region with joining segment) to a second DNA segment encoding at least a part of human constant region. Each fused gene is assembled in or inserted into an expression vector. Recipient cells capable of expressing the gene products are then transfected with the genes. The transfected recipient cells are cultured under conditions that permit expression of the incorporated genes and the expressed immunoglobulins or immunoglobulin chains are recovered.

Genes encoding the variable region of immunoglobulin heavy and light chains are obtained from lymphoid cells that produce the monoclonal rabies virus neutralizing antibodies. For example, the heterohybridoma cell lines that produce monoclonal antibody against the rabies glycoprotein provide a source of immunoglobulin variable region for the present chimeric antibodies. Constant regions are obtained from human antibody-producing cells by standard cloning techniques. Alternatively, because genes are representing the two classes of light chains an the give classes of heavy chains have been cloned, constant regions of human origin are readily available from these clones. Chimeric antibody binding fragments such as F(ab').sub.2 and Fab fragments are prepared by designing a chimeric heavy chain gene in truncated form. For example, a chimeric gene encoding a F(ab')₂ heavy chain portion would include DNA sequences encoding the CH₁ domain and hinge region of the heavy chain. Alternatively, such fragments can be obtained by enzymatic cleavage of a chimeric immunoglobulin. For instance, papain or pepsin cleavage can generate Fab or F(ab')₂ fragments, respectively.

Preferably, the fused genes encoding the heavy and light chimeric chains, or portions thereof, are assembled into two different expression vectors that can be used to cotransfect a recipient cell. Each vector contains two selectable genes, one for selection in a bacterial system, and one for selection in a eukaryotic system, each vector having a different pair of genes. These vectors allow production and amplification of the fused genes in bacterial system, and subsequent cotransfection of eukaryotic cells and selection of the cotransfected cells. Examples of selectable genes for the bacterial system include, but are not limited to, the genes that confer ampicillin resistance and the gene that confers chloramphenicol resistance. Two selectable genes for selection of eukaryotic transfectants are preferred, but are not limited to: (i) the xanthine-guanine phosphoribosyltransferase gene (gpt), and (ii) the phosphotransferase gene from Tn5 (designated neo). Selection with gpt is based on the ability of the enzyme encoded by this gene to use xanthine as a substrate for purine nucleotide synthesis; the analogous endogenous enzyme cannot. In a medium containing xanthine and mycophenolic acid, which blocks the conversion of inosine monophosphate to xanthine monosphosphate, only cells expressing the gpt gene can survive. The product of the neo blocks the inhibition of protein synthesis in eukaryotic cells caused by the antibiotic G418 and other antibiotics of its class. The two selection procedures can be used simultaneously or sequentially to select for the expression of immunoglobulin chain genes introduced on two different DNA vectors into a eukaryotic cell.

### Expression systems

Due to the inherent degeneracy of the genetic code, also described herein are other DNA sequences which encode substantially the same or a functionally equivalent heavy and light chain amino acid sequences. Altered DNA sequences which may be used include deletions, additions or substitutions of different nucleotide residues resulting in a sequence that encodes the same, or a functionally equivalent, gene product. The gene product itself may contain deletions, additions or substitutions of amino acid residues within a heavy or light chain sequence which result in a silent change, thus producing a functionally equivalent monoclonal antibody.

Nucleotide sequences coding for heavy and light chains of the monoclonal rabies virus neutralizing antibody, a fragment or analog thereof, are inserted into an appropriate expression vector. This vector which contains the necessary elements for transcription and translation of the inserted protein-coding sequence so as to generate recombinant DNA molecules that direct the expression of heavy and light chain immunoglobulins for the formation of monoclonal rabies virus neutralizing antibody.

The preferred recipient cell line is a myeloma cell. Myeloma cells can synthesize, assemble and secrete immunoglobulins encoded by transfected immunoglobulin genes. Further, they possess the mechanism for glycosylation of the immunoglobulin. A particularly preferred recipient cell is a myeloma cell line that does not produce immunoglobulin, such as Sp2/0. These cell lines produce only the immunoglobulin encoded by the transfected immunoglobulin genes. Myeloma cells can be grown in culture or in the peritoneum of mice where secreted immunoglobulin can be obtained from ascites fluid. Other lymphoid cells such as B lymphocytes or hybridoma cells can serve as suitable recipient cells.

Several methods exist for transfecting lymphoid cells with vectors containing immunoglobulin encoding genes. A preferred way of introducing DNA into lymphoid cells is by electroporation. In this procedure recipient cells are subjected to an electric pulse in the presence of the DNA to be incorporated. Another way to introduce DNA is by protoplast fusion. In this method, lysozyme is used to strip cell walls from bacteria harboring the recombinant plasmid containing the immunoglobulin gene. The resulting spheroplasts are fused with myeloma cells with polyethylene glycol. After protoplast fusion, the transfectants are selected and isolated. Another technique that can be used to introduce DNA into may cell types is calcium phosphate precipitation.

The immunoglobulin genes can also be expressed in nonlymphoid cells, such as bacteria or yeast. When expressed in bacteria, the immunoglobulin heavy chains and light chains become part of inclusion bodies. Thus, the chains must be isolated and purified and then assembled into functional immunoglobulin molecules. Other strategies for expression in *E. coli* are available (see e.g., Pluckthun, A., BioTechnology 9:545-551, 1991; Skerra, A. et al., BioTechnology 9:273-278,1991), including secretion from *E.coli* as fusion proteins comprising a signal sequence.

### Example 2

The entire sequence of two monoclonal antibodies against the rabies virus, MAb 57 and MAb JB.1 were determined. The monoclonal antibodies bind specifically to the glycoprotein of various rabies virus strains. Post-exposure treatment, as well as prophylactic treatment, with a cocktail of monoclonal antibodies neutralizes the rabies virus at the site of entry and prevents the virus from spreading to the central nervous system (CNS). Thus, for transdermal or mucosal exposure to rabies virus, a cocktail of rabies specific-monoclonal antibodies are instilled into the bite site, as well as administered systemically. Since viral replication is restricted almost exclusively to neuronal cells, neutralization and clearance of the virus by the monoclonal antibodies of the present invention prior to entry into the CNS is an effective post-exposure prophylaxis.

A cocktail of monoclonal antibodies against rabies virus is delivered to the patient that has been exposed, or is at high risk of exposure, to rabies virus. The cocktail of monoclonal antibodies of the present invention effectively inhibits the formation of any rabies variants that can escape neutralization, as each monoclonal antibody in the cocktail of monoclonal antibodies has specificity for an epitope that is conserved in different street rabies viruses.

The nucleotide sequence of human anti-rabies MAb JB.1 heavy chain is SEQ ID NO:9. The amino acid sequence of human anti-rabies MAb JB.1 heavy chain is SEQ ID NO:10. The nucleotide sequence of human anti-rabies MAb JB.1 light chain is SEQ ID NO:11. The amino acid sequence of human anti-rabies MAb JB.1 light chain is SEQ ID NO: 12. The nucleotide sequence of human anti-rabies MAb 57 light chain is SEQ ID NO: 13. The amino acid sequence of human anti-rabies MAb 57 light chain is SEQ ID NO:14. The nucleotide sequence of human anti-rabies MAb 57 heavy chain is SEQ ID NO:15. The amino acid sequence of human anti-rabies MAb 57 heavy chain is SEQ ID NO:16.

## Claims

1. An isolated nucleic acid encoding an antibody composed of an antibody heavy chain and an antibody light chain, said nucleic acid comprising a first nucleic acid segment encoding the antibody heavy chain having the amino acid sequence SEQ ID NO: 10, and a second nucleic acid segment encoding the antibody light chain having the amino acid sequence SEQ ID NO:12.

2. The isolated nucleic acid of claim 1, wherein the first nucleic acid segment has the nucleotide sequence SEQ ID NO:9 and the second nucleic acid segment has the nucleotide sequence SEQ ID NO: 11.

3. A recombinant expression vector comprising an isolated nucleic acid according to claim 1 or 2.

4. A host cell comprising an expression vector according to claim 3.

5. A method of producing an isolated recombinant antibody comprising culturing a host cell according to claim 4 and isolating recombinant antibodies expressed thereby.

6. A recombinant antibody comprising a heavy chain having the amino acid sequence SEQ ID NO:10 and a light chain having the amino acid sequence SEQ ID NO:12.

7. A recombinant antibody comprising:
(a) a chimeric immunoglobulin heavy chain encoded by a fused gene comprising:
(i) a first DNA sequence encoding the variable region of the antibody heavy chain polypeptide having the amino acid sequence SEQ ID NO:10; and
(ii) a second DNA sequence encoding a human heavy chain constant region; and
(b) a chimeric immunoglobulin light chain encoded by a fused gene comprising:
(i) a first DNA sequence encoding the variable region of the antibody light chain polypeptide having the amino acid sequence SEQ ID NO: 12; and
(ii) a second DNA sequence encoding a human light chain constant region.

8. An Fv, Fab or F(ab')₂ fragment of an antibody comprising a heavy chain having the amino acid sequence SEQ ID NO:10 and a light chain having the amino acid sequence SEQ ID NO: 12, which antibody fragment has rabies virus-neutralizing activity.

9. An antibody fragment containing the six complement determining regions of the antibody comprising a heavy chain having the amino acid sequence SEQ ID NO: 10 and a light chain having the amino acid sequence SEQ ID NO:12, which antibody fragment has rabies virus-neutralizing activity.

10. An antibody according to claim 6 or 7 for use as a medicament.

11. An antibody fragment according to claim 8 or 9 for use as a medicament.

12. An antibody according to claim 6 or 7 for use in treating an individual exposed to a rabies virus.

13. An antibody fragment according to claim 8 or 9 for use in treating an individual exposed to a rabies virus.

## Patentansprüche

1. Eine isolierte Nukleinsäure, die einen Antikörper codiert, der aus einer schweren Kette des Antikörpers und einer leichten Kette des Antikörpers zusammengesetzt ist, wobei die Nukleinsäure ein erstes Nukleinsäuresegment, das die schwere Kette des Antikörpers mit der Aminosäuresequenz SEQ ID NO: 10 codiert und ein zweites Nukleinsäuresegment, das die leichte Kette des Antikörpers mit der Aminosäuresequenz SEQ ID NO: 12 codiert, beinhaltet.

2. Isolierte Nukleinsäure gemäß Anspruch 1, wobei das erste Nukleinsäuresegment die Nukleotidsequenz SEQ ID NO: 9 aufweist und das zweite Nukleinsäuresegment die Nukleotidsequenz SEQ ID NO: 11 aufweist.

3. Ein rekombinanter Expressionsvektor, der eine isolierte Nukleinsäure gemäß Anspruch 1 oder 2 beinhaltet.

4. Eine Wirtszelle, die einen Expressionsvektor gemäß Anspruch 3 beinhaltet.

5. Ein Verfahren zum Herstellen eines isolierten rekombinanten Antikörpers, das das Kultivieren einer Wirtszelle gemäß Anspruch 4 und das Isolieren von dadurch exprimierten rekombinanten Antikörpern beinhaltet.

6. Ein rekombinanter Antikörper, der eine schwere Kette mit der Aminosäuresequenz SEQ ID NO: 10 und eine leichte Kette mit der Aminosäuresequenz SEQ ID NO: 12 beinhaltet.

7. Ein rekombinanter Antikörper, der Folgendes beinhaltet:
(a) eine schwere Kette des chimären Immunglobulins, die von einem fusionierten Gen codiert ist, das Folgendes beinhaltet:
(i) eine erste DNA-Sequenz, die den variablen Bereich einer schweren Kette eines Antikörper-Polypeptids mit der Aminosäuresequenz SEQ ID NO: 10 codiert; und
(ii) eine zweite DNA-Sequenz, die einen konstanten Bereich einer menschlichen schweren Kette codiert; und
(b) eine leichte Kette des chimären Immunglobulins, die von einem fusionierten Gen codiert ist, das Folgendes beinhaltet:
(i) eine erste DNA-Sequenz, die den variablen Bereich einer leichten Kette des Antikörper-Polypeptids mit der Aminosäuresequenz SEQ ID NO: 12 codiert; und
(ii) eine zweite DNA-Sequenz, die einen konstanten Bereich einer menschlichen leichten Kette codiert.

8. Ein Fv-, Fab- oder F(ab')₂-Fragment eines Antikörpers, der eine schwere Kette mit der Aminosäuresequenz SEQ ID NO: 10 und eine leichte Kette mit der Aminosäuresequenz SEQ ID NO: 12 beinhaltet, wobei das Antikörperfragment eine das Tollwutvirus neutralisierende Aktivität aufweist.

9. Ein Antikörperfragment, das die sechs komplementbestimmenden Bereiche des Antikörpers, der eine schwere Kette mit der Aminosäuresequenz SEQ ID NO: 10 und eine leichte Kette mit der Aminosäuresequenz SEQ ID NO: 12 beinhaltet, enthält, wobei das Antikörperfragment eine das Tollwutvirus neutralisierende Aktivität aufweist.

10. Antikörper gemäß Anspruch 6 oder 7 zur Verwendung als ein Medikament.

11. Antikörperfragment gemäß Anspruch 8 oder 9 zur Verwendung als ein Medikament.

12. Antikörper gemäß Anspruch 6 oder 7 zur Verwendung beim Behandeln eines einem Tollwutvirus ausgesetzten Individuums.

13. Antikörperfragment gemäß Anspruch 8 oder 9 zur Verwendung beim Behandeln eines einem Tollwutvirus ausgesetzten Individuums.

## Revendications

1. Un acide nucléique isolé codant pour un anticorps composé d'une chaîne lourde d'anticorps et d'une chaîne légère d'anticorps, ledit acide nucléique comprenant un premier segment d'acide nucléique codant pour la chaîne lourde d'anticorps ayant la séquence d'acides aminés SEQ ID NO : 10, et un deuxième segment d'acide nucléique codant pour la chaîne légère d'anticorps ayant la séquence d'acides aminés SEQ ID NO : 12.

2. L'acide nucléique isolé de la revendication 1, dans lequel le premier segment d'acide nucléique a la séquence nucléotidique SEQ ID NO : 9 et le deuxième segment d'acide nucléique a la séquence nucléotidique SEQ ID NO : 11.

3. Un vecteur d'expression recombiné comprenant un acide nucléique isolé selon la revendication 1 ou la revendication 2.

4. Une cellule hôte comprenant un vecteur d'expression selon la revendication 3.

5. Une méthode pour produire un anticorps recombiné isolé comprenant cultiver une cellule hôte selon la revendication 4 et isoler des anticorps recombinés exprimés par celle-ci.

6. Un anticorps recombiné comprenant une chaîne lourde ayant la séquence d'acides aminés SEQ ID NO : 10 et une chaîne légère ayant la séquence d'acides aminés SEQ ID NO : 12.

7. Un anticorps recombiné comprenant :
(a) une chaîne lourde d'immunoglobuline chimérique codée par un gène fusionné comprenant :
(i) une première séquence d'ADN codant pour la région variable du polypeptide à chaîne lourde d'anticorps ayant la séquence d'acides aminés SEQ ID NO : 10 ; et
(ii) une deuxième séquence d'ADN codant pour une région constante de chaîne lourde humaine ; et
(b) une chaîne légère d'immunoglobuline chimérique codée par un gène fusionné comprenant :
(i) une première séquence d'ADN codant pour la région variable du polypeptide à chaîne légère d'anticorps ayant la séquence d'acides aminés SEQ ID NO : 12 ; et
(ii) une deuxième séquence d'ADN codant pour une région constante de chaîne légère humaine.

8. Un fragment Fv, Fab ou F(ab')₂ d'un anticorps comprenant une chaîne lourde ayant la séquence d'acides aminés SEQ ID NO : 10 et une chaîne légère ayant la séquence d'acides aminés SEQ ID NO : 12, lequel fragment d'anticorps a une activité de neutralisation du virus de la rage.

9. Un fragment d'anticorps contenant les six régions de détermination de complément de l'anticorps comprenant une chaîne lourde ayant la séquence d'acides aminés SEQ ID NO : 10 et une chaîne légère ayant la séquence d'acides aminés SEQ ID NO : 12, lequel fragment d'anticorps a une activité de neutralisation du virus de la rage.

10. Un anticorps selon la revendication 6 ou la revendication 7 destiné à être utilisé comme médicament.

11. Un fragment d'anticorps selon la revendication 8 ou la revendication 9 destiné à être utilisé comme médicament.

12. Un anticorps selon la revendication 6 ou la revendication 7 destiné à être utilisé dans le traitement d'un individu exposé à un virus de la rage.

13. Un fragment d'anticorps selon la revendication 8 ou la revendication 9 destiné à être utilisé dans le traitement d'un individu exposé à un virus de la rage.
